# EUROPÄISCHE PATENTSCHRIFT

(11) **EP 2 855 650 B1**
(45) Veröffentlichungstag und Bekanntmachung des Hinweises auf die Patenterteilung: **28.12.2016**
(21) Anmeldenummer: 13725692.1
(22) Anmeldetag: 29.05.2013
(51) Int. Cl.: C11D 1/94, A61K 8/42, A61Q 5/02, A61Q 19/10, A61K 8/37, A61K 8/41, A61K 8/44, A61K 8/46, A61K 8/92, C11D 1/02, C11D 1/14, C11D 3/20, C11D 3/43

(54) **N-METHYL-N-ACYLGLUCAMIN ENTHALTENDE ZUSAMMENSETZUNG**
N-METHYL-N-ACYLGLUCAMINE-CONTAINING COMPOSITION
COMPOSITION CONTENANT DE LA N-MÉTHYL-N-ACYLGLUCAMINE

(30) Priorität: 30.05.2012 DE 102012010656
(43) Veröffentlichungstag der Anmeldung: 08.04.2015
(73) Patentinhaber: Clariant International Ltd, 4132 Muttenz (CH)
(72) Erfinder: KLUG, Peter, 63762 Großostheim (DE); MILDNER, Carina, 65931 Frankfurt am Main (DE)
(74) Vertreter: Holmes, Rosalind
(86) Internationale Anmeldenummer: PCT/EP2013/061075
(87) Internationale Veröffentlichungsnummer: WO 2013/178683

(56) Entgegenhaltungen:
- EP-A1- 1 078 978
- WO-A1-92/06161
- WO-A1-93/19149
- WO-A1-96/03974

## Beschreibung

Die Erfindung betrifft eine Zusammensetzung, enthaltend mindestens ein anionisches Tensid, ein Betaintensid, ein N-Methyl-N-acylglucamin, ein Triglyceridöl, ein Lösungsmittel und gegebenenfalls ein Additiv, sowie ein Verfahren zur Herstellung der Zusammensetzung. Ferner betrifft die Erfindung die Verwendung der Zusammensetzung zur Behandlung oder Pflege der Haut oder Haare oder als Shampoo, Gesichtsreiniger, Flüssigreiniger oder Duschbad.

Bei der Herstellung von Zusammensetzungen, insbesondere zur Herstellung von Reinigungszusammensetzungen, sollten eine Reihe von Kriterien beachtet werden, wie beispielsweise eine gute reinigende Wirkung, ausreichende schäumende Eigenschaften, gute Hautverträglichkeit und ein gutes Gefühl in Bezug auf Haut und Haare und speziell keine Reizung der Schleimhäute. Haut und Haare bestehen aus mehren Schichten, die unter anderem Keratin und Kollagen als Faserproteine umfassen. Anionische Tenside können in die Schichten eindringen und diese zerstören. Ideale kosmetische Reinigungsmittel für kosmetische oder pharmazeutische Anwendungen sollten die Haut oder das Haar sanft reinigen, ohne Entfettung und/oder Trocknen der Haare und der Haut und ohne zu reizen.

Bei der Herstellung kosmetischer oder dermatologischer Zubereitungen tritt häufig das Problem auf, dass bestimmte Inhaltsstoffe, insbesondere Pflegekomponenten wie kosmetische Öle, keine ausreichende Wasserlöslichkeit aufweisen und die Zubereitungen, insbesondere in Gegenwart von Salzen, trüb werden oder mehrere Phasen bilden.

WO 95/17880 betrifft Shampoozusammensetzungen mit funktionellen Materialien als Konditionierungsmittel, Stylingmittel oder Antischuppenmittel. Dabei wird eine leichte, gut schäumende Shampoozusammensetzung mit hoher Abscheidung von funktionellen Materialien bereitgestellt, wobei die Shampoozusammensetzung umfasst: (a) etwa 5% bis etwa 40 Gew.% Tensid umfassend: (i) 80% bis etwa 99%, bezogen auf das Tensidsystem, anionische Tenside, die Alkyl-ethoxylierte Sulfate und Alkylsulfate in einem Verhältnis zwischen etwa 1:1 und 1:0 sind, und (ii) etwa 1% bis 20%, bezogen auf das Tensidsystem, Polyhydroxyfettsäureamid-Tenside, (b) von etwa 0,05% bis etwa 25 Gew.% funktionelle Materialien, und (c) etwa 35% bis etwa 95 Gew.% Wasser.

WO 96/37592 beschreibt ein mildes, schaumproduzierendes Reinigungsprodukt für die Reinigung der Haut oder der Haare, wobei dieses als Schaumbad und Duschprodukt, Hautreinigungsmittel und Shampoo verwendet werden kann. Gemäß einem Aspekt dieser Schrift wird ein Waschmittel, Reinigungsmittel oder kosmetische Zusammensetzung bereitgestellt, umfassend: (A) etwa 1% bis etwa 25 Gew.% wasserlösliches Gel, welches ein nichtionischen Tensids bildet; (B) etwa 0,1% bis etwa 3 Gew.% eines Alkylsulfats als Fluidisierungsmittel, welches im Durchschnitt 4 bis 10 Kohlenstoffatomen in der Alkylkette aufweist; und (C) gegebenenfalls von etwa 1% bis etwa 30 Gew.% einer dispergierten Ölphase. Die Tenside bilden dabei homogene (opake) Produkt-Matrizen, in denen die Öl-Tröpfchen einen Durchmesser im Bereich von etwa 1 Mikrometer bis etwa 30 Mikrometer aufweisen.

Das Dokument WO 92/06158 betrifft eine gering schäumende Waschmittelzusammensetzung, umfassend mindestens 1 Gew.-% eines Polyhydroxyfettsäureamid-Tensids der Formel 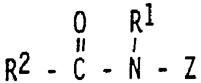 worin R¹ H, C₁-C₄-Kohlenwasserstoff, 2-Hydroxyethyl oder 2-Hydroxypropyl ist, R² C₅-C₃₁-Kohlenwasserstoff ist und Z ein Polyhydroxykohlenwasserstoff mit einer linearen Kohlenwasserstoffkette mit mindestens drei direkt mit der Kette verbundenen Hydroxylgruppen oder alkoxylierte Derivate davon ist; mindestens 1% eines alkylalkoxylierten Sulfat-Tensids; und wahlweise eine schaumunterdrückende Menge eines Schaumunterdrückers, welcher aus der monocarboxylische Fettsäuren und Salze davon, Silikon-Schaumunterdrücker und Monostearylkiakalimetallphosphaten oder-phosphatester und hochmolekulargewichtige Kohlenwasserstoff-Schaumunterdrücker und Mischungen hiervon umfassenden Gruppe gewählt ist; wobei die Zusammensetzung ein alkylalkoxyliertes Sulfat:Polyhydroxyfettsäureamid-Gewichtsverhältnis von 10:1 bis 1:10, vorzugsweise 5:1 bis 1:5 und stärker bevorzugt 4:1 bis 1:1 aufweist und das Polyhydroxyfettsäureamid weniger als 4 Gew.-% eines cyclischen Amid-Nebenprodukts umfasst.

Hinsichtlich der Formulierung von kosmetischen Mitteln, insbesondere Haarpflegemitteln, weisen Fettsäure-N-alkylglucamide jedoch den Nachteil auf, dass ihr Schaumvermögen nicht immer zufriedenstellend ist. Dies bezieht sich einerseits auf die Höhe des Basisschaums, andererseits auch auf die Schaumbeständigkeit, insbesondere in hartem Wasser. Ein weiterer Nachteil besteht darin, dass die Amide nach dem Abspülen oftmals ein stumpfes Gefühl auf den Haaren hinterlassen und die Kämmbarkeit tendenziell eher verschlechtern.

Die Aufgabe der Erfindung liegt somit darin, eine verbesserte Zusammensetzung bereitzustellen, die hohe Mengen an natürlichen Ölen als rückfettende Substanz erlaubt und zudem klar ist.

Demgemäß wird eine klare Zusammensetzung bereitgestellt, enthaltend:
(A) mindestens ein anionisches Tensid als Komponente A,
(B) mindestens ein Betaintensid als Komponente B,
(C) mindestens ein N-Methyl-N-acylglucamin als Komponente C, wobei das N-Methyl-N-acylglucamin einen C₈-C₂₂-Acylrest aufweist,
(D) mindestens ein Triglyceridöl als Komponente D,
(E) mindestens ein Lösungsmittel als Komponente E und
(F) gegebenenfalls ein oder mehrere Additive als Komponente F.

Durch die erfindungsgemäße Zusammensetzung lassen sich hohe Mengen an natürlichen Ölen einbringen. Zudem muss die Zusammensetzung nicht gegen eine Separation stabilisiert werden. Überraschenderweise weisen die erfindungsgemäßen Zusammensetzungen trotz der Gegenwart von Triglyceridölen einen sehr guten Schaumaufbau auf. Im Vergleich zu konventionellen Ethersulfat/Betainsystemen oder entsprechenden Formulierungen aus Basis Alkylpolyglucosid sind die Zusammensetzungen klar und phasenstabil und weisen eine gute Rückfettungsleistung auf.

Erfindungsgemäß ist die Zusammensetzung klar. Insbesondere kann darunter verstanden werden, dass die Zusammensetzung in Schichtdicken von 5 cm optisch durchsichtig ist und nicht opak und emulsionsartig erscheint, wie beispielsweise Zusammensetzungen aus dem genannten Stand der Technik. Weiterhin weisen die Zusammensetzungen keine Separation in mehrere Phasen auf und sind damit homogen.

Weitere Begriffe für N-Methyl-N-acylglucamin sind N-Methyl-N-1-Desoxysorbitol-Fettsäureamid, N-Acyl-N-methylglucamin, Glucamid oder N-Methyl-N-alkylglucamid. Dabei entspricht N-Methyl-N-acylglucamin der Formel (X), wobei R ein organischer Rest ist:

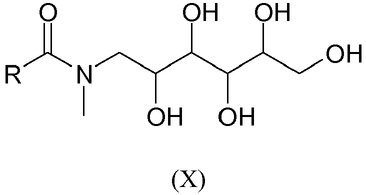

In einer bevorzugten Ausführungsform ist die Komponente A ausgewählt aus einer oder mehreren Verbindung(en) der allgemeinen Formel (I),

R¹SO₃⁻ M+ (I)

wobei R¹ für Alkyl, Cycloalkyl, Aralkyl, Aryl, Alkoxy, Alkoxyalkyl und Heterocyclyl steht und M+ ein Alkalimetall-, Erdalkalimetall- oder ein substituiertes oder nicht substituiertes Ammoniumion ist, oder
der allgemeinen Formel (II),

R¹SO₄⁻ M+ (II)

wobei R¹ für Alkyl, Cycloalkyl, Aralkyl, Aryl, Alkoxy, Alkoxyalkyl und Heterocyclyl steht und M+ ein Alkalimetall-, Erdalkalimetall- oder ein substituiertes oder nicht substituiertes Ammoniumion ist.

"Alkyl" bedeutet eine gesättigte aliphatische Kohlenwasserstoffgruppe, die geradkettig oder verzweigt sein kann und von 1 bis 20 Kohlenstoffatome in der Kette haben kann. Bevorzugte Alkylgruppen können geradkettig oder verzweigt sein und von 1 bis zu 10 Kohlenstoffatome in der Kette aufweisen. Verzweigt bedeutet, dass eine NiederAlkylgruppe, wie Methyl, Ethyl oder Propyl, an eine lineare Alkylkette angebracht ist. Bei Alkyl handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Di-methyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl und 1-Octadecyl. "Cycloalkyl" bedeutet einen aliphatischen Ring, der von 3 bis 10 Kohlenstoffatome in dem Ring hat. Bevorzugte Cycloalkylgruppen haben von 4 bis 7 Kohlenstoffatome in dem Ring.

"Aryl" bedeutet Phenyl oder Naphthyl.

"Aralkyl" bedeutet eine Alkylgruppe, die mit einem Arylrest substituiert ist.

"Substituiertes Aralkyl" und "substituiertes Aryl" bedeuten, dass die Arylgruppe oder die Alkylgruppe der Aralkylgruppe mit einem oder mehreren Substituenten ausgewählt aus Alkyl, Alkoxy, Nitro, Carboalkoxy, Cyano, Halo, Alkylmercaptyl, Trihaloalkyl oder Carboxyalkyl substituiert ist.

"Alkoxy" bedeutet eine Alkyl-O-Gruppe, in der "Alkyl" die vorstehend beschriebene Bedeutung hat. Nieder-Alkoxy-Gruppen sind bevorzugt. Beispiele sind Methoxy, Ethoxy, n-Propoxy, i-Propoxy und n-Butoxy.

"Nieder-Alkyl" bedeutet eine Alkylgruppe, die 1 bis 7 Kohlenstoffatome aufweist. "Alkoxyalkyl" bedeutet eine Alkylgruppe wie vorstehend beschrieben, die mit einer Alkoxygruppe, wie vorstehend beschrieben, substituiert ist. Somit kann unter dem Begriff Alkoxyalkyl ein Polyether verstanden werden.

"Heterocyclyl" bedeutet eine 4 bis 10-gliedrige Ringstruktur, in der ein oder mehrere Ringatome von Kohlenstoff verschieden sind, beispielsweise N, O oder S sind. Heterocyclyl kann aromatisch oder nicht-aromatisch sein, d. h. es kann gesättigt, teilweise oder ganz ungesättigt sein.

Im Rahmen einer bevorzugten Ausführungsform ist das anionische Tensid der Komponente A ein Alkylsulfat oder ein Alkylethersulfat.

Besonders bevorzugt sind Natriumlaurylsulfat und/oder Natriumlaurylethersulfat.

Im Rahmen einer bevorzugten Ausführungsform umfasst die Komponente B mindestens ein Alkylbetain und/ oder mindestens ein Alkylamidobetain.

Beispiele für geeignete Alkylbetaine stellen die Carboxyalkylierungsprodukte von Aminen, insbesondere sekundären und tertiären Aminen der Formel (III) dar 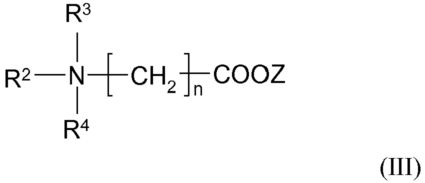 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Wasserstoff oder Alkylreste mit 1 bis 4 Kohlenstoffatomen, n für Zahlen von 1 bis 6 und Z für ein Alkaliund/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C12/14-Kokosalkyldimethylamin, Myristyldimethylamin, Cetyldimethylamin, Stearyldimethylamin, Stearylethylmethylamin, Oleyldimethylamin, C16/18-Talgalkyldimethylamin sowie deren technische Gemische.

Beispiele für geeignete Alkylamidobetaine stellen Carboxyalkylierungsprodukte von Amidoaminen da. Insbesondere geeignet sind Amidopropylbetaine der Formel (IV), 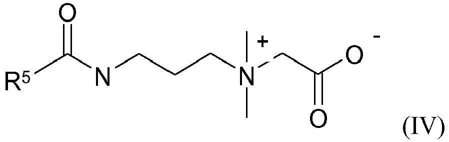 worin R⁵ eine lineare oder verzweigte gesättigte C₇-C₂₁-Alkylgruppe oder eine lineare oder verzweigte ein- oder mehrfach ungesättigte C₇-C₂₁-Alkenylgruppe ist.

Bevorzugten Betaintenside sind Amidopropylbetaine wie Cocoamidopropylbetain (R⁵CO ist der Fettsäurerest des Kokosöls, Kettenlänge C₈-C₁₈) und Alkylbetaine wie Coco-Betain (R² ist der Alkylrest des Kokosöls, Kettenlänge C₈-C₁₈) oder Laurylbetain (R² ist ein Alkylrest der Kettenlänge C₁₂ und C₁₄)

Unter einem Lösungsmittel im Rahmen der Erfindung versteht man vorzugsweise protische Lösungsmittel wie Wasser, C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt. Bevorzugtes Lösungsmittel ist Wasser.

Als Triglycerid kommen beispielweise Triglyceride von linearen oder verzweigten, gesättigten oder ungesättigten, gegebenenfalls hydroxylierten C₈-C₃₀-Fettsäuren in Betracht, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-Pfirsichkern-, Makadamia-, Avocado-, Süßmandel-, Wiesenschaumkraut-, Ricinus-, Oliven-, Erdnuss-, Raps- und Kokosnussöl, sowie synthetische Triglyceridöle, z. B. das Handelsprodukt Myritol® 318. Im wesentlichen ungesättigte Triglyceride pflanzlichen Ursprungs wie Olivenöl sind erfindungsgemäß bevorzugt. Auch Öle tierischen Ursprungs, beispielweise Rindertalg, Perhydrosqualen, und Lanolin können eingesetzt werden.

Bevorzugt sind Triglyceride, die C₈-C₂₂-Fettsäuren enthalten, besonders bevorzugt C₈-C₁₈-Fettsäuren, insbesondere gesättigte oder ungesättigte C₁₆-C₁₈-Fettsäuren.

Besonders bevorzugt sind Triglyceride, die im wesentlichen C₁₆- und C₁₈- Fettsäuren (sogenannte C16/C18-Fettsäuren) enthalten, wie beispielsweise Sonnenblumenöl, Mandelöl und Olivenöl.

Im Rahmen einer bevorzugten Ausführungsform enthält die Zusammensetzung
(A) 5 - 15 Gew.-% der Komponente A,
(B) 1 - 4 Gew.-% der Komponente B,
(C) 0,5 - 5,0 Gew.-% der Komponente C,
(D) 0,01-3,0 Gew.-% der Komponente D,
(E) 5 - 93,49 Gew.-% eines protischen Lösungsmittels als Komponente E und
(F) 0 - 10 Gew.-% der Komponente F,
wobei die Summe der Komponenten A bis F 100 Gew.-% ergibt.

Bevorzugt besteht die Zusammensetzung aus
(A) 5 - 15 Gew.-% der Komponente A,
(B) 1 - 4 Gew.-% der Komponente B,
(C) 0,5 - 5,0 Gew.-% der Komponente C,
(D) 0,01 - 3,0 Gew.-% der Komponente D,
(E) 5 - 93,49 Gew.-% eines protischen Lösungsmittels als Komponente E und
(F) 0 - 10 Gew.-% der Komponente F,
wobei die Summe der Komponenten A bis F 100 Gew.-% ergibt.

Besonders geeignet ist ein Triglyceridölgehalt von 0,05-2,0 Gew.-%, vorzugsweise von 0,1-1,0 Gew.-% und insbesondere von 0,2-0,6 Gew.-%, wobei die Summe aller Komponenten A bis F 100 Gew.-% ergibt.

### Im Rahmen einer weiteren bevorzugten Ausführungsform enthält die Zusammensetzung (und besteht insbesondere aus)

(A) 7,0 - 10,0 Gew.-% der Komponente A,
(B) 1,5 - 3,4 Gew.-% der Komponente B,
(C) 1,0 - 5,0 Gew.-% der Komponente C,
(D) 0,01 - 3,0 Gew.-% der Komponente D,
(E) 5 - 90,49 Gew.-% eines protischen Lösungsmittels als Komponente E und
(F) 0 - 10 Gew.-% der Komponente F,
wobei die Summe der Komponenten A bis F 100 Gew.-% ergibt.

Im Rahmen einer bevorzugten Ausführungsform ist der Acylrest in der Komponente C aus der Gruppe der linearen, der verzweigten, der gesättigten, der ungesättigten C₈-C₂₂-Acylreste oder Mischungen daraus ausgewählt. Besonders bevorzugt ist ein C₈-C₁₈-Acylrest. Dabei ist beispielsweise unter einem C₈- Acylrest ein von Octansäure abgeleiteter Acylrest zu verstehen.

Im Rahmen einer bevorzugten Ausführungsform beträgt die Summe der Komponenten A, B und C von 6,5 - 24,0 Gew.-%, bevorzugt von 10 bis 18 Gew.-% und insbesondere von 11 bis 17 Gew.-%.

Im Rahmen einer bevorzugten Ausführungsform besteht die Komponente C aus einem oder einer Mischung von mehreren gesättigten oder ungesättigten N-Methyl-N-acylglucaminen.

Im Rahmen einer bevorzugten Ausführungsform ist das Lösungsmittel Wasser oder ein Gemisch aus Wasser und Propylenglykol.

Im Rahmen einer bevorzugten Ausführungsform sind die Additive aus der Gruppe bestehend aus Konservierungsmitteln, Duftstoffen, Farbstoffen, weiteren Tensiden, Wasser, Ölkörpern, kationische Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus ausgewählt, bevorzugt in Mengen von 0,1 bis 10,0 Gew.-%, besonders bevorzugt von 0,5 bis 8,0 Gew.-% und insbesondere von 1,0 bis 5,0 Gew.-%.

Als Konservierungsmittel eignen sich die im betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard^{®} DMDMH).

Als Duft- bzw. Parfümstoffe oder Öle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die gewünschte Viskosität der Zusammensetzungen kann durch Zugabe von Verdickern und Gelierungsmittel eingestellt (erhöht oder ernidriegt) werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammonium Acryloyldimethyltaurate/ Craboxyethylacrylate Crosspolymer (Aristoflex TAC®, Clariant) Verwendung finden.

Besonders gut geeignet als Verdicker ist Kochsalz.

An antimikrobiellen Wirkstoffen kommen beispielsweise Cetyltrimethylammoniumchlorid, Cetyl-pyridiniumchlorid, Benzethoniumchlorid, Diisobutylethoxyethyl-dimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergika, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Als Stabilisatoren können Metallsalze von Fettsäuren, wie z. B. Magnesium-, Aluminium- und/oder Zinkstearat eingesetzt werden.

Als feuchtigkeitsspendende Substanz stehen beispielsweise Isopropylpalmitat, Glycerin und/oder Sorbitol zu Verfügung.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Zitronensäure, verwendet.

Im Rahmen einer bevorzugten Ausführungsform handelt es sich bei der Zusammensetzung um eine kosmetische, dermatologische oder pharmazeutische Zusammensetzung.

Ein weiterer Gegenstand der Erfindung ist ein Verfahren zur Herstellung der erfindungsgemäßen Zusammensetzung, umfassend die Schritte:
a) Mischen der Komponenten A und D,
b) Zugabe der Komponenten B und E und anschließendes Homogenisieren der Komponenten A,B,D und E,
c) Zugabe von der Komponente C in flüssiger Form und anschließendes Homogenisieren der Komponenten A, B, C, D und E,
d) gegebenenfalls Zugabe von Komponente F und
e) Einstellen des pH-Werts der Zusammensetzung auf 5-8.

Vorzugsweise umfasst das Verfahren die vorstehend genannten Mengenangaben der erfindungsgemäßen Zusammensetzung.

Ein weiterer Gegenstand der Erfindung ist die Verwendung der erfindungsgemäßen Zusammensetzung als Shampoo, Gesichtsreiniger, Flüssigreiniger oder Duschbad.

Ein weiterer Gegenstand der Erfindung ist die Verwendung der erfindungsgemäßen Zusammensetzung zur Behandlung oder Pflege der Haut.

Ein weiterer Gegenstand der Erfindung ist die Verwendung der erfindungsgemäßen Zusammensetzung zur Behandlung oder Pflege der Haare.

Die Erfindung wird durch die nachstehenden Beispiele näher erläutert:
Herstellbeispiel H1 bis H3, Beispiele 1 bis 5 sowie Vergleichsbeispiele 1 und 2.

Die im Folgenden beschriebenen N-Acyl-N-methyl-glucamine wurden nach EP 0 550 637 aus den korrespondierenden Fettsäuremethylestern und N-Methylglucamin in Gegenwart von 1,2 Propylenglykol als Lösemittel hergestellt und als Feststoff bestehend aus Aktivsubstanz und 1,2 Propylenglykol erhalten.

C12/14 bedeutet, dass der Methylester aus einem Gemisch aus Laurinsäuremethylester ( C₁₂-Acylrest) und Myristinsäuremethylester (C₁₄-Acylrest) besteht (Verhältnis 75 : 25). C8/C14 bedeutet, dass der Methylester aus einem Gemisch aus Octansäuremethylester, Decansäuremethylester, Laurinsäuremethylester und Myristinsäuremethylester (Verhältnis 7 : 8 : 64 : 21) besteht. C16/18 bedeutet, dass der Methylester aus einem Gemisch aus Palmitinsäuremethylester (C₁₆-Acylrest) und Stearinsäuremethylester (C₁₈-Acylrest) besteht (Verhältnis 30 : 70). C12/18 bedeutet, dass der Methylester aus einem Gemisch aus Laurinsäuremethylester, Myristinsäuremethylester, Palmitinsäuremethylester, Stearinsäuremethylester und Ölsäuremethylester (Verhältnis 64 : 21 : 5 : 2 : 8) besteht.

**Tabelle 1**

| Herstellbeispiel | Methylester | Aktivsubstanz (%) | 1,2 Propylenglykol (%) | Schmelzpunkt |
|---|---|---|---|---|
| H1 | C12/14 | 90 | 10 | 85 |
| H2 | C 8/14 | 90 | 10 | 70 |
| H3 | C 16/18 | 80 | 20 | 68 |
| H4 | C12/18 | 90 | 10 | 75 |

Die Viskositäten wurden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa•s, Spindel 2 für Viskositäten von maximal 1 000 mPa•s, Spindel 3 für Viskositäten von maximal 5 000 mPa•s, Spindel 4 für Viskositäten von maximal 10 000 mPa • s, Spindel 5 für Viskositäten von maximal 20 000 mPa•s, Spindel 6 für Viskositäten von maximal 50 000 mPa•s und Spindel 7 für Viskositäten von maximal 200 000 mPa•s gewählt.

**Testformulierung:**

| | | |
|---|---|---|
| Triglyceridölhaltige Formulierungen wurden anhand der folgenden allgemeinen Rezeptur erstellt: | | |
| Komponente A | Genapol^{®} LRO flüssig (*Natriumlaurylethersulfat)* | |
| (27 % Lösung in Wasser, berechnet auf 100 % Aktivsubstanz) | | 7,2 - 9,6 Gew.-% |
| Komponente D | Triglyceridöl | 0,2 - 0,6 Gew-% |
| Komponente B | Genagen^{®} CAB 818 (*Cocamidopropylbetaine*) | |
| (30 % Lösung in Wasser, berechnet auf 100 % Aktivsubstanz) | | 2,4 Gew.-% |
| Komponente E | Wasser | ad 100,0 Gew.-% |
| Komponente C | Zuckertensid nach Herstellbeispiel H1 -H3 | 2,67 bzw. 3 Gew.-% |
| Komponente F | Konservierungsmittel (Nipaguard^{®}DMDMH) | 0,2 Gew.-% |

| | | |
|---|---|---|
| (ad 100,0 bedeutet ein Auffüllen auf insgesamt 100, 00 Gew.-%, wobei die Komponenten C und F in den 100 % eingeschlossen sind). | | |

### Herstellung:

Genapol^{®} LRO liquid und Triglyceridöl wurden bei 25 °C gemischt. Wasser und Cocamidopropylbetain wurden bei 25 °C zugegeben und homogenisiert. Aufgeschmolzenes Zuckertensid wurde zugegeben, bei 75 °C homogenisiert und anschließend durch Zugabe von Kochsalz die Viskosität anpasst. Das Konservierungsmittel wurde nach dem Abkühlen auf Raumtemperatur zugegeben und der pH-Wert mit Zitronensäure oder NaOH auf pH = 5,5 anpasst. Das Aussehen der Formulierungen wurde nach 4 Tagen bewertet.

Alle Gehaltsangaben beziehen sich auf Aktivgehalt des Tensids.

**Tabelle 2**

| Beispiel | Genapol® LRO flüssig (%) | Cocamidopropylbetain (%) | Triglyceridöl | Anteil Triglyceridöl (%) | Zuckertensid | Aussehen der Formulierung | NaCl Zugabe (%) | Viskosität (mPas) |
|---|---|---|---|---|---|---|---|---|
| Vergleichsbeispiel 1 | 9,6 | 2,4 | Olivenöl | 0,5 | - | weiß, trüb | 2,2 | 4750 |
| Vergleichsbeispiel 2 | 7,2 | 2,4 | Olivenöl | 0,5 | Coco-Glucosid (Plantacare 818) | weiß, trüb | 2,5 | 7200 |
| 1 | 7,2 | 2,4 | Olivenöl | 0,5 | Herstellbeispiel H1 | klar | 2,0 | 4700 |
| 2 | 7,2 | 2,4 | Olivenöl | 0,5 | Herstellbeispiel H2 | klar | 2,8 | 5100 |
| 3 | 7,2 | 2,4 | Olivenöl | 0,5 | Herstellbeispiel H3 | klar | 2,4 | 4900 |
| 4 | 7,2 | 2,4 | Olivenöl | 0,2 | Herstellbeispiel H1 | klar | 1,9 | 4600 |
| 5 | 7,2 | 2,4 | Sonnenblumenöl | 0,4 | Herstellbeispiel H1 | klar | 1,9 | 4750 |
| 6 | 7,2 | 2,4 | Olivenöl | 0,5 | Herstellbeispiel H 4 | klar | 1,6 | 5200 |

Aus den erfindungsgemäßen Beispielen 1-6 ist zu erkennen, dass durch den Einsatz der erfindungsgemäßen N-Acyl-N-methyl-glucamine klare, verdickbare, triglyceridölhaltige Formulierungen erhalten werden können. Dagegen lassen sich ohne Zuckertensid (Vergleichsbeispiel 1) oder mit anderen Zuckertensiden wie Alkylpolyglucosiden keine klaren Formulierungen erhalten.

Analoge Testformulierungen, die mit Silikonöl (Dimethicon 50 cSt) hergestellt wurden, ergaben ebenfalls keine klaren Formulierungen.

## Patentansprüche

1. Klare Zusammensetzung, enthaltend:
(A) mindestens ein anionisches Tensid als Komponente A,
(B) mindestens ein Betaintensid als Komponente B,
(C) mindestens ein N-Methyl-N-acylglucamin als Komponente C, wobei das N-Methyl-N-acylglucamin einen C₈-C₂₂-Acylrest aufweist,
(D) mindestens ein Triglyceridöl als Komponente D,
(E) mindestens ein Lösungsmittel als Komponente E und
(F) gegebenenfalls ein oder mehrere Additive als Komponente F.

2. Zusammensetzung nach Anspruch 1, enthaltend:
(A) 5 - 15 Gew.-% der Komponente A,
(B) 1 - 4 Gew.-% der Komponente B,
(C) 0,5 - 5 Gew.-% der Komponente C,
(D) 0,01 - 3,0 Gew.-% der Komponente D
(E) 5 - 93,49 Gew.-% eines protischen Lösungsmittels als der Komponente E und
(F) 0 - 10 Gew.-% der Komponente F,
wobei die Summe der Komponenten A bis F 100 Gew.-% ergibt.

3. Zusammensatzung nach Anspruch 1 oder 2, **dadurch gekennzeichnet, dass** die Komponente A ausgewählt ist aus einer oder mehreren Verbindung(en) der allgemeinen Formel (I),
R¹SO₃⁻ M⁺ (I)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist, oder
der allgemeinen Formel (II),
R¹SO₄⁻ M⁺ (II)
wobei R¹ für Alkyl, Cycloalkyl, Aralkyl, Aryl, Alkoxy, Alkoxyalkyl und Heterocyclyl steht und M⁺ ein Alkalimetall-, Erdalkalimetall- oder ein substituiertes oder nicht substituiertes Ammoniumion ist.

4. Zusammensetzung nach einem der Ansprüche 1 bis 3, **dadurch gekennzeichnet, dass** das anionische Tensid der Komponente A ein Alkylsulfat oder ein Alkylethersulfat ist.

5. Zusammensetzung nach einem der Ansprüche 1 bis 4, **dadurch gekennzeichnet, dass** die Komponente B mindestens ein Alkylbetain und/ oder mindestens ein Alkylamidobetain umfasst.

6. Zusammensetzung nach einem der Ansprüche 1 bis 5, **dadurch gekennzeichnet, dass** der Acylrest in der Komponente C aus der Gruppe der linearen, der verzweigten, der gesättigten, der ungesättigten C₈-C₂₂-Acylreste oder Mischungen daraus ausgewählt ist.

7. Zusammensetzung nach einem der Ansprüche 2 bis 6, **dadurch gekennzeichnet, dass** die Summe der Komponenten A, B und C von 6,5 bis 24 Gew.-% beträgt.

8. Zusammensetzung nach einem der Ansprüche 1 bis 7, **dadurch gekennzeichnet, dass** Komponente C aus einem oder einer Mischung von mehreren gesättigten oder ungesättigten N-Methyl-N-acylglucaminen besteht.

9. Zusammensetzung nach einem der Ansprüche 1 bis 8, **dadurch gekennzeichnet, dass** das Lösungsmittel Wasser oder ein Gemisch aus Wasser und Propylenglykol ist.

10. Zusammensetzung nach einem der Ansprüche 1 bis 9, **dadurch gekennzeichnet, dass** die Additive aus der Gruppe bestehend aus Konservierungsmitteln, Duftstoffen, Farbstoffen, weiteren Tensiden, Wasser, Ölkörpern, kationische Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus ausgewählt sind.

11. Zusammensetzung nach einem der Ansprüche 1 bis 10, **dadurch gekennzeichnet, dass** es sich bei der Zusammensetzung um eine kosmetische, dermatologische oder pharmazeutische Zusammensetzung handelt.

12. Verfahren zur Herstellung der Zusammensetzung nach einem der Ansprüche 1 bis 11, umfassend die Schritte:
a) Mischen der Komponenten A und D,
b) Zugabe der Komponenten B und E und anschließendes Homogenisieren der Komponenten A,B,D und E,
c) Zugabe von der Komponente C in flüssiger Form und anschließendes Homogenisieren der Komponenten A, B, C, D und E,
d) gegebenenfalls Zugabe von Komponente F und
e) Einstellen des pH-Werts der Zusammensetzung auf 5-8.

13. Verwendung der Zusammensetzung nach einem der Ansprüche 1 bis 11 als Shampoo, Gesichtsreiniger, Flüssigreiniger oder Duschbad.

14. Nicht therapeutische Verwendung der Zusammensetzung nach einem der Ansprüche 1 bis 11 zur Behandlung oder Pflege der Haut.

15. Verwendung der Zusammensetzung nach einem der Ansprüche 1 bis 11 zur Behandlung oder Pflege der Haare.

## Claims

1. Clear composition comprising:
(A) at least one anionic surfactant as component A,
(B) at least one betaine surfactant as component B,
(C) at least one N-methyl-N-acylglucamine as component C, wherein the N-methyl-N-acylgluamine has a C₈-C₂₂-acyl radical,
(D) at least one triglyceride oil as component D,
(E) at least one solvent as component E, and
(F) optionally one or more additives as component F.

2. Composition according to Claim 1, comprising:
(A) from 5 to 15% by weight of component A,
(B) from 1 to 4% by weight of component B,
(C) from 0.5 to 5% by weight of component C,
(D) from 0.01 to 3.0% by weight of component D,
(E) from 5 to 93.49% by weight of a protic solvent as component E, and
(F) from 0 to 10% by weight of component F, wherein the sum of components A to F is 100% by weight.

3. Composition according to Claim 1 or 2, **characterized in that** component A is selected from one or more compound(s) of general formula (I)
R¹SO₃⁻ M⁺ (I)
wherein R¹ represents alkyl, cycloalkyl, aralkyl, aryl, alkoxy, alkoxyalkyl and heterocyclyl and M⁺ is an alkali metal, alkaline earth metal or substituted or unsubstituted ammonium ion, or
of general formula (II)
R¹SO₄⁻M⁺ (II)
wherein R¹ represents alkyl, cycloalkyl, aralkyl, aryl, alkoxy, alkoxyalkyl and heterocyclyl and M⁺ is an alkali metal, alkaline earth metal or substituted or unsubstituted ammonium ion.

4. Composition according to one of Claims 1 to 3, **characterized in that** the anionic surfactant of component A is an alkyl sulphate or an alkyl ether sulphate.

5. Composition according to one of Claims 1 to 4, **characterized in that** component B comprises at least one alkyl betaine and/or at least one alkylamido betaine.

6. Composition according to one of Claims 1 to 5, **characterized in that** the acyl radical in component C is selected from the group of linear, branched, saturated or unsaturated C₈-C₂₂-acyl radicals or mixtures thereof.

7. Composition according to one of Claims 2 to 6, **characterized in that** the sum of components A, B and C is from 6.5 to 24% by weight.

8. Composition according to one of Claims 1 to 7, **characterized in that** component C consists of one or a mixture of two or more saturated or unsaturated N-methyl-N-acylglucamines.

9. Composition according to one of Claims 1 to 8, **characterized in that** the solvent is water or a mixture of water and propylene glycol.

10. Composition according to one of Claims 1 to 9, **characterized in that** the additives are selected from the group consisting of preservatives, fragrances, dyes, further surfactants, water, oily substances, cationic polymers, film-forming agents, thickeners and gelling agents, superfatting agents, antimicrobial and biogenic active ingredients, moisture-donating agents, stabilizers, acids, lyes, activity enhancers, and mixtures thereof.

11. Composition according to one of Claims 1 to 10, **characterized in that** the composition is a cosmetic, dermatological or pharmaceutical composition.

12. Method for preparing the composition according to one of Claims 1 to 11, comprising the steps of:
a) mixing components A and D,
b) adding components B and E and then homogenising components A, B, D and E,
c) adding component C in liquid form and then homogenising components A, B, C, D and E,
d) optionally adding component F and
e) adjusting the pH of the composition to 5-8.

13. Use of the composition according to one of Claims 1 to 11 as a shampoo, facial cleanser, liquid cleanser or shower gel.

14. Non-therapeutic use of the composition according to one of Claims 1 to 11 for the treatment or care of the skin.

15. Use of the composition according to one of Claims 1 to 11 for the treatment or care of the hair.

## Revendications

1. Composition transparente contenant :
(A) au moins un tensioactif anionique en tant que composant A,
(B) au moins un tensioactif de type bétaïne en tant que composant B,
(C) au moins une N-méthyl-N-acylglucamine en tant que composant C, la N-méthyl-N-acylglucamine comportant un radical acyle en C₈-C₂₂,
(D) au moins une huile de triglycéride en tant que composant D,
(E) au moins un solvant en tant que composant E et
(F) éventuellement un ou plusieurs additifs en tant que composant F.

2. Composition selon la revendication 1, contenant :
(A) 5-15 % en poids du composant A,
(B) 1-4 % en poids du composant B,
(C) 0,5-5 % en poids du composant C,
(D) 0,01-3,0 % en poids du composant D,
(E) 5-93,49 % en poids d'un solvant protique en tant que composant E, et
(F) 0-10 % en poids du composant F,
la somme des composants A à F étant égale à 100 % en poids.

3. Composition selon la revendication 1 ou 2, **caractérisée en ce que** le composant A est choisi parmi un ou plusieurs composé(s) de formule générale (I),
R¹SO₃⁻ M⁺ (I)
dans laquelle R¹ représente un groupe alkyle, cycloalkyle, aralkyle, aryle, alcoxy, alcoxyalkyle et hétérocyclyle et M⁺ est un ion de métal alcalin, de métal alcalino-terreux ou un ion ammonium substitué ou non substitué, ou
de formule générale (II),
R¹SO₄⁻ M⁺ (II)
dans laquelle R¹ représente un groupe alkyle, cycloalkyle, aralkyle, aryle, alcoxy, alcoxyalkyle et hétérocyclyle et M⁺ est un ion de métal alcalin, de métal alcalino-terreux ou un ion ammonium substitué ou non substitué.

4. Composition selon l'une quelconque des revendications 1 à 3, **caractérisée en ce que** le tensioactif anionique du composant A est un alkylsulfate ou un alkyléthersulfate.

5. Composition selon l'une quelconque des revendications 1 à 4, **caractérisée en ce que** le composant B comprend au moins une alkylbétaïne et/ou au moins une alkylamidobétaïne.

6. Composition selon l'une quelconque des revendications 1 à 5, **caractérisée en ce que** le radical acyle dans le composant C est choisi dans le groupe des radicaux acyle en C₈-C₂₂ linéaires, des radicaux acyle en C₈-C₂₂ ramifiés, des radicaux acyle en C₈-C₂₂ saturés, des radicaux acyle en C₈-C₂₂ insaturés ou des mélanges de ceux-ci.

7. Composition selon l'une quelconque des revendications 2 à 6, **caractérisée en ce que** la somme des composants A, B et C vaut de 6,5 à 24 % en poids.

8. Composition selon l'une quelconque des revendications 1 à 7, **caractérisée en ce que** le composant C consiste en une N-méthyl-N-acylglucamine saturée ou insaturée ou en un mélange de plusieurs N-méthyl-N-acylglucamines saturées ou insaturées.

9. Composition selon l'une quelconque des revendications 1 à 8, **caractérisée en ce que** le solvant est l'eau ou un mélange d'eau et de propylèneglycol.

10. Composition selon l'une quelconque des revendications 1 à 9, **caractérisée en ce que** les additifs sont choisis dans le groupe constitué par des conservateurs, des parfums, des colorants, d'autres tensioactifs, l'eau, des corps huileux, des polymères cationiques, des agents filmogènes, des épaississants et gélifiants, des agents de regraissage, des substances actives antimicrobiennes et biogènes, des agents hydratants, des stabilisants, des acides, des solutions alcalines, des activateurs et des mélanges de ceux-ci.

11. Composition selon l'une quelconque des revendications 1 à 10, **caractérisée en ce que** pour ce qui est de la composition il s'agit d'une composition cosmétique, dermatologique ou pharmaceutique.

12. Procédé pour la préparation de la composition selon l'une quelconque des revendications 1 à 11, comprenant les étapes :
a) mélange des composants A et D,
b) addition des composants B et E et homogénéisation subséquente des composants A, B, D et E,
c) addition du composant C sous forme liquide et homogénéisation subséquente des composants A, B, C, D et E,
d) éventuellement addition du composant F et
e) ajustement du pH de la composition à 5-8.

13. Utilisation de la composition selon l'une quelconque des revendications 1 à 11, en tant que shampooing, nettoyant pour le visage, produit de nettoyage liquide ou gel douche.

14. Utilisation non thérapeutique de la composition selon l'une quelconque des revendications 1 à 11, pour le traitement ou le soin de la peau.

15. Utilisation de la composition selon l'une quelconque des revendications 1 à 11, pour le traitement ou le soin des cheveux.
